(19) Europäisches Patentamt
European Patent Office
Office européen des brevets

(11) EP 1 519 909 B1

(12) EUROPÄISCHE PATENTSCHRIFT

(45) Veröffentlichungstag und Bekanntmachung des Hinweises auf die Patenterteilung:
**01.12.2010 Patentblatt 2010/48**

(21) Anmeldenummer: **03740270.8**

(22) Anmeldetag: **18.06.2003**

(51) Int Cl.:
***C07C 57/48*** *(2006.01)* ***C07C 59/48*** *(2006.01)*
***C12P 7/44*** *(2006.01)*

(86) Internationale Anmeldenummer:
**PCT/EP2003/006407**

(87) Internationale Veröffentlichungsnummer:
**WO 2004/005236 (15.01.2004 Gazette 2004/03)**

(54) **POLYENCARBONSÄURE-DERIVATE, VERFAHREN ZU IHRER HERSTELLUNG UND IHRE VERWENDUNG**

POLYENE CARBOXYLIC ACID DERIVATIVES, METHOD FOR THEIR PRODUCTION AND THE USE THEREOF

DERIVES D'ACIDE POLYENECARBOXYLIQUE, PROCEDE POUR LES PRODUIRE ET LEUR UTILISATION

(84) Benannte Vertragsstaaten:
**AT BE BG CH CY CZ DE DK EE ES FI FR GB GR HU IE IT LI LU MC NL PT RO SE SI SK TR**

(30) Priorität: **02.07.2002 DE 10229713**

(43) Veröffentlichungstag der Anmeldung:
**06.04.2005 Patentblatt 2005/14**

(73) Patentinhaber: **Sanofi-Aventis Deutschland GmbH**
**65929 Frankfurt am Main (DE)**

(72) Erfinder:
- **VERTESY, Lászlo**
  **65817 Eppstein-Vockenhausen (DE)**
- **KURZ, Michael**
  **65830 Kriftel (DE)**
- **WINK, Joachim**
  **63322 Rödermark (DE)**

(56) Entgegenhaltungen:
- **M RITZAU ET AL.: "Metabolic Products of Microorganisms. 266. Serpentene, a Novel Polyene Carboxylic Acid from Streptomyces" LIEBIGS ANNALEN DER CHEMIE, Nr. 4, 1993, Seiten 433-435, XP0009017577 in der Anmeldung erwähnt**
- **N DARBY ET AL.: "Synthesis of Benzannelated Bisdehydro [14]-, -[16]-, -[18]-, and -[20] annulenes" JOURNAL OF ORGANIC CHEMISTRY, Bd. 42, Nr. 11, 1977, Seiten 1960-1967, XP002254975 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 519 909 B1

## Beschreibung

Zur Behandlung von bakteriellen Infektionskrankheiten wird eine große Zahl von Antibiotika therapeutisch eingesetzt. Die Krankheitserreger werden aber zunehmend resistent gegen die verwendeten Arzneimittel, und es droht sogar eine große Gefahr durch sogenannte multiresistente Keime, die nicht nur gegen einzelne Antibiotikagruppen, wie z.B. β-Lactam-Antibiotika, Glycopeptide oder Macrolide widerstandsfähig geworden sind, sondern gleichzeitig mehrere Resistenzen tragen. Es gibt sogar Krankheitserreger, die gegen alle im Handel erhältlichen Antibiotika resistent geworden sind. Infektionskrankheiten, die durch solche Keime verursacht werden, sind nicht mehr therapierbar. Deshalb gibt es einen großen Bedarf an neuen Mitteln, die gegen resistente Keime eingesetzt werden können. Es sind zwar in der Literatur viele Tausend Antibiotika beschrieben worden, die meisten sind jedoch zu toxisch, um als Arzneimittel eingesetzt werden zu können.

Es wurde bereits eine größere Anzahl von Polyen-Antibiotika beschrieben, von denen die meisten zum makrocyclischen Strukturtypus gehören. Diese Makrolide wirken antimykotisch durch Wechselwirkungen mit biologischen Membranen und sind daher gegen Warmblüter toxisch. Der wichtigste Vertreter diesen Typs ist das Amphotericin B, das trotz seiner Toxizität als Humantherapeutikum eingesetzt wird. Als nichtmakrocyclisches Polyen-Antibiotikum wurde zum Beispiel das Serpenten (Ritzau et al., Liebigs Ann. Chem. 1993, 433-435) beschrieben, das einen in 1,2-Position mit Polyenseitenketten substituierten Phenylring enthält. Serpenten zeigte in Tests gegen Gram-positive und Gram-negative Bakterien ausschliesslich gegen Bacillus subtilis eine schwache antibiotische Wirkung.

Die Zellwand von Gram-positiven Bakterien ist unter anderem aus Murein aufgebaut, das aus N-Acetyl-D-Glukosamin und N-Acetylmuraminsäure, die disaccharidähnlich untereinander verbunden sind, aufgebaut ist und Aminosäuren sowie Peptide wie zum Beispiel D-Glutamin, D- und L-Alanin, L-Lysin und Pentaglycyl-Einheiten enthält, und das stark vernetzt ist. Die Biosynthese der bakteriellen Zellwand geschieht mit Hilfe von Enzymen, die im Stoffwechsel von Warmblütern nicht vorkommen. Daher sind solche Enzyme geeignete Angriffsorte für die Entwicklung von für Warmblüter gut verträglichen Antibiotika, und Inhibitoren der Mureinbiosynthese sollten für Menschen ungiftig sein. Ein Schlüsselenzym Peptidoglycanbiosynthese, und damit des Zellwandaufbaus, ist die Glycosyltransferase (Transglycosylase, GT). Ein spezifischer Inhibitor dieses Enzyms, das Moenomycin (Kurz et al., Eur. J. Biochem. 1998, 252, 500-507), ist bereits seit längerer Zeit bekannt. Das Moenomycin ist ein sehr stark wirksames Antibiotikum, welches gut verträglich ist, es wird jedoch aus dem Magen-Darm-Trakt nicht resorbiert und auch die Ausscheidung aus dem Blut nach intravenöser Gabe ist gestört, so daß eine systemische Anwendung in der Medizin bislang unterbleiben musste. Es sind später nur sehr wenige weitere Glycosyltransferase-Inhibitoren in der Literatur beschrieben worden, aus Gründen der Pharmakokinetik oder der Verträglichkeit fand keines dieser Agenzien den Weg in die Therapie. Es werden daher neuartige GT-Inhibitoren weiterhin gesucht, wie es in neuerer Zeit von Goldman & Gange in Current Medicinal Chem. 2000, 7, 801-820, berichtet wurde. Das Screening geschieht mit spezifischen biochemischen GT-Testsystemen. Solche Assays sind wiederholt beschrieben wurden, wie z.B. von Vollmer & Höltje in Antimicrobial Agents and Chemotherapy 2000, 44, 1181-1185.

Darby et al. (J. Org. Chem. 1977, 42, 1960-1967) beschreiben die Synthese von in 1,2-Position mit trans-Polyenseitenketten substituierte Phenylverbindungen zur Untersuchung des π-Systems von makrozyklischen Annulenen.

Es wurde überraschend gefunden, daß der Stamm *Actinomycetales* sp. DSM 14865 neuartige Verbindungen zu bilden vermag, die sehr gute Inhibitoren der Glycosyltransferase und sehr wirksame antibakterielle Verbindungen sind.

Gegenstand der Erfindung sind demzufolge die von dem Stamm, *Actinomycetales* sp. DSM 14865, gebildeten Wirkstoffe sowie deren physiologisch verträglichen Salze. Gegenstand der Erfindung ist demgemäß eine Verbindung der Formel (IV)

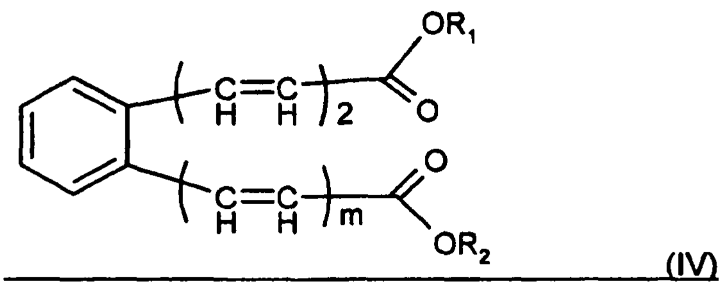

(IV)

wobei

$R_1$ gleich H oder $C_1$-$C_6$-Alkyl,

$R_2$ gleich H oder $C_1$-$C_6$-Alkyl, und

m 3 oder 4 sind, sowie deren Physiologisch verträgliche Salze.

$C_1$-$C_6$-Alkyl bedeutet eine geradkettige oder verzweigte Alkylgruppe mit 1 bis 6 C-Atomen, bevorzugt mit 1 bis 4 C-Atomen, wie z.B. Methyl, Ethyl, i-Propyl, tert.Butyl und Hexyl.

Die Konfiguration der Doppelbindungen in den Polyengruppen der Verbindungen der Formel (I) kann, wenn nicht anders angegeben, in der *cis*- oder in der *trans*-Konfiguration vorliegen. Die Erfindung betrifft sowohl die reinen Verbindungen als auch Stereoisomerengemische, wie Enantiomerengemische und Diasteromerengemische, in jedem Verhältnis. Unter stereoisomeren Formen werden insbesondere Verbindungen mit unterschiedlichen räumlichen Anordnungen (Konfigurationen) der Atome oder Atomgruppen in einem Molekül bei gleichartiger Verkettung der Atome verstanden, zum Beispiel *cis-trans* Isomere an Doppelbindungen.

Vorzugsweise enthält mindestens eine Polyengruppe in der Verbindung der Formel (I) mindestens eine *cis*-Doppelbindung.

Besonders bevorzugt betrifft die Erfindung eine Verbindung der Formel (IV), in der m gleich 4 ist, und in denen die Konfiguration der Polyene der Formel (V) entspricht:

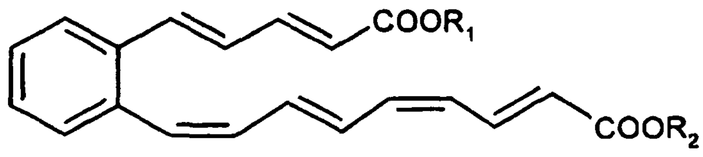

(V)

Speziell bevorzugt ist eine Verbindung der Formel (V), in der $R_1$ und $R_2$ gleich H sind. Diese Verbindung wird im folgenden als Serpentemycin A (Summelformel: $C_{20}H_{18}O_4$; MG = 322.36) bezeichnet.

Ferner besonders bevorzugt betrifft die Erfindung eine Verbindung der Formel (IV), in der n gleich 2 und m gleich 3 ist, und in denen die Konfiguration der Polyene der Formel (VI) entspricht:

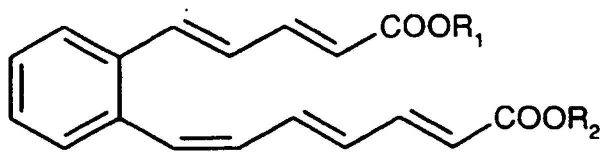

(VI)

Speziell bevorzugt ist eine Verbindung der Formel (VI), in der $R_1$ und $R_2$ gleich H sind. Diese Verbindung wird im folgenden als Serpentemycin B (Summelformel: $C_{18}H_{16}O_4$; MG = 296.33) bezeichnet.

Weiterhin besonders bevorzugt betrifft die Erfindung eine Verbindung der Formel (IV), in der n gleich 2 und m gleich 3 ist, und in denen die Konfiguration der Polyene der Formel (VII) entspricht:

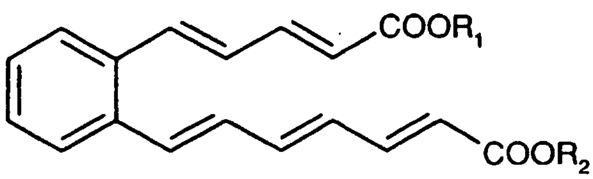

(VII)

Speziell bevorzugt ist eine Verbindung der Formel (VII), in der $R_1$ und $R_2$ gleich H sind. Diese Verbindung wird im folgenden als Serpentemycin C (Summelformel: $C_{18}H_{16}O_4$; MG = 296.33) bezeichnet.

Bevorzugt betrifft die Erfindung eine Verbindung der Formel (I), gekennzeichnet durch eine Verbindung der Formel (VIII)

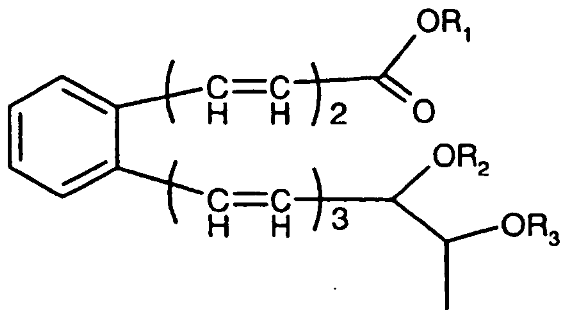

(VIII),

sowie deren physiologisch verträglichen Salze.

Die Erfindung betrifft ferner eine Verbindung der Summenformel $C_{20}H_{18}O_4$ (Serpentemycin A) oder; $C_{18}H_{16}O_4$ (Serpentemycin) erhältlich durch Fermentierung von *Actinomycetales* sp. DSM 14865 oder einer ihrer Varianten und/oder Mutanten in einem Kulturmedium bis sich die Verbindungen Serpentemycin A, und/oder B in der Kulturlösung anhäuft, und durch anschließende Isolierung der Verbindung, sowie gegebenenfalls Überführung in ein pharmakologisch verträgliches Salz.

Die Erfindung betrifft eine Verbindung der Formel (I), erhältlich durch Fermentierung von *Actinomycetales* sp. DSM 14865 bzw. einer ihrer Varianten und/oder Mutanten in einem Kulturmedium, bis sich eine oder mehrere der Verbindungen Serpentemycin A und/oder B in der Kulturbrühe anhäuft, anschließende Isolierung der Verbindung, gegebenenenfalls Überführung in ein chemisches Derivat, sowie gegebenenfalls Überführung in ein pharmakologisch verträgliches Salz.

Durch die angegebenen Strukturformeln unterscheiden sich die Serpentemycine A und B von literaturbekannten Substanzen. Es sind zwar strukturverwandte Polyencarbonsäure-Derivate bekannt, sie unterscheiden sich jedoch durch ihre chemische Struktur, die antimikrobielle beziehungsweise biochemische Wirksamkeit und durch weitere physikalischen Eigenschaften von den erfindungsgemäßen Verbindungen.

Weiterhin betrifft die Erfindung ein Verfahren zur Herstellung der Verbindung der Formel (I), dadurch gekennzeichnet, daß

1. der Mikroorganismus *Actinomycetales* sp. DSM 14865 bzw. einer ihrer Varianten und/oder Mutanten in einem wäßrigen Nährmedium kultiviert wird, bis sich eine oder mehrere der Verbindungen Serpentemycin A und/oder B in der Kulturbrühe anhäuft,
2. Serpentemycin A oder B isoliert und gereinigt wird,
3. Serpentemycin A oder B gegebenenfalls mit einem geeigneten Reagenz in eine Verbindung der Formel (I) überführt wird,
4. und die Verbindung der Formel (I) gegebenenfalls in ein pharmakologisch verträgliches Salz überführt wird.

Geeignete Reagenzien sind beispielsweise Alkylierungsmittel, mit denen Carboxylgruppen der Serpentemycine A und B zu dem entsprechenden Ester umgesetzt werden können. Alkylierende Agenzien sind zum Beispiel Methyljodid, Diethylsulfat, Diazomethan oder andere Alkyl-Derivate, wie sie beispielsweise von Jerry March im Buch Advanced Organic Chemistry, John Wiley & Sons, 4th Edition, 1992, beschrieben worden sind. Doppelbindungen können beispielsweise photochemisch oder unter Zusatz eines Radikalbildners isomerisiert werden. Um Umsetzungen selektiv durchzuführen, kann es vorteilhaft sein vor der Reaktion geeignete, in an sich bekannter Weise, Schutzgruppen einzuführen. Die Schutzgruppen werden nach der Reaktion abgespaltet und anschließend wird das Reaktionsprodukt gereinigt.

Eine Verbindung der Formel (I) kann nach dem Fachmann bekannten Methoden in die entsprechenden pharmakologisch verträglichen Salze übergeführt werden. Unter pharmakologisch verträglichen Salzen der erfindungsgemäßen Verbindungen versteht man sowohl anorganische als auch organische Salze, wie sie in Remingtons Pharmaceutical Sciences (17. Auflage, Seite 1418 [1985]) beschrieben sind. Als Salze kommen insbesondere Alkali-, Ammonium-, Erdalkalisalze, Salze mit physiologisch verträglichen Aminen und Salze mit anorganischen oder organischen Säuren wie z. B. HCl, HBr, $H_2SO_4$, Maleinsäure, Fumarsäure in Frage.

Der Stamm *Actinomycetales* sp. DSM 14865 bildet auf Glukose-, Stärke- Hefeextrakt- oder Glycerin-haltigen Nährlösungen die Verbindungen Serpentemycine A und B.

*Actinomycetales* sp. DSM 14865 bildet darüberhinaus zahlreiche Nebenprodukte mit geringfügig variierter Polyenketten.

Ein Isolat von *Actinomycetales* sp. wurde bei der Deutschen Sammlung von Mikroorganismen und Zellkulturen

GmbH (DSM), Mascheroder Weg 1 B, 38124 Braunschweig, Deutschland nach den Regeln des Budapester Vertrages am 18.03.2002 unter der Nummer DSM 14865 hinterlegt.

*Actinomycetales* sp. DSM 14865 besitzt ein braunbeiges Substratmycel und spärliches Luftmycel auf Haferflockenmedium. Er bildet in Kultur keine für die *Actinomyceten* charakteristischen Fruchtkörper.

Das besagte Verfahren umfasst die Kultivierung von *Actinomycetales* sp. DSM 14865, seiner Mutanten und/ oder Varianten unter aeroben Bedingungen in eine Kohlenstoff- und Stickstoffquelle, anorganischen Salze und gegebenensfalls Spurenelemente enthaltenden Kulturmedien.

Anstelle des Stammes *Actinomycetales* sp. DSM 14865 können auch deren Mutanten und Varianten eingesetzt werden, soweit sie die erfindungsgemäßen Verbindungen synthetisieren.

Eine Mutante ist ein Mikroorganismus, in dem ein oder mehrere Gene des Genoms modifiziert wurden, wobei das Gen oder die Gene funktionell und vererbbar erhalten bleiben, die für die Fähigkeit des Organismus verantwortlich sind, die erfinderische Verbindung zu produzieren.

Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS); 2-Hydroxy-4-methoxy-benzophenon (MOB) oder N-Methyl-N'-nitro-N-nitrosoguanidin (MNNG) erzeugt werden, oder wie von Brock et al. in "Biology of Microorganisms", Prentice Hall, Seite 238-247 (1984) beschrieben.

Eine Variante ist ein Phenotyp des Mikroorganismus. Mikroorganismen haben die Fähigkeit, sich an ihre Umgebung anzupassen und zeigen daher ausgeprägte physiologische Flexibilität. Bei der phenotypischen Anpassung sind alle Zellen des Mikroorganismus involviert, wobei die Art der Veränderung nicht genetisch konditioniert ist und unter veränderten Bedingungen reversibel ist (H. Stolp, Microbial ecology: organismus, habitats, activities. Cambridge University Press, Cambridge, GB, Seite 180, 1988).

Das Screening nach Mutanten und Varianten, die das erfindungsgemäße Antibiotikum produzieren, kann durch Bestimmung der biologischen Aktivität des in der Kulturbrühe angehäuften Wirkstoffes, beispielsweise durch Bestimmung der antibakteriellen Wirkung erfolgen, oder durch Detektion von Verbindungen, die als antibakteriell aktiv bekannt sind, in der Fermentationsbrühe durch beispielsweise HPLC-oder LC-MS-Methoden.

Als bevorzugte Kohlenstoffquellen für die Fermentation eignen sich assimilierbare Kohlenhydrate und Zukkeralkohole, wie Glukose, Laktose, Saccharose oder D-Mannit sowie kohlenhydrathaltige Naturprodukte, wie z.B. Malzextrakt oder Hefextrakt. Als Stickstoffquelle kommen in Betracht: Aminosäuren, Peptide und Proteine sowie deren Abbauprodukte, wie Casein, Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate, insbesondere aber auch synthetisch bzw. biosynthetisch gewonnene Peptide. An anorganischen Salzen und Spurenelemente kann die Nährlösung beispielsweise Chloride, Carbonate, Sulfate oder Phosphate der Alkali- oder Erdalkalimetalle, Eisen, Zink, Kobalt und Mangan enthalten.

Die Bildung der erfindungsgemäßen Verbindungen verläuft besonders gut z.B. in einer Nährlösung, die etwa 0,05 bis 5 %, bevorzugt 0,5 bis 2 % Stärke, 0,05 bis 3 %, bevorzugt 0.1 bis 1 % Hefeextrakt, 0,05 bis 5 %, bevorzugt 0,1 bis 2 % Glucose, 0,2 bis 5 %, bevorzugt 0.5% bis 2 % Glycerin, 0,05 bis 3 %, bevorzugt 0,1 bis 1 % Consteep, 0,05 bis 3 %, bevorzugt 0,1 bis 1 % Pepton, 0,05 bis 3 %, bevorzugt 0,05 bis 0,5 % NaCl und 0,05 bis 3 %, bevorzugt 0,1 bis 1 % $CaCO_3$ enthält. Die Angaben in Prozent sind jeweils bezogen auf das Gewicht der gesamten Nährlösung.

In der Nährlösung bildet *Actinomycetales* sp. DSM 14865 ein Gemisch aus den Serpetemycinen A und B und Nebenprodukten. Je nach Zusammensetzung der Nährlösung kann der mengenmäßige Anteil einer oder mehrerer der erfindungsgemäßen Verbindungen variieren. Außerdem kann durch die Medienzusammensetzung die Synthese einzelner Polyencarbonsäuren gesteuert werden, so daß ein Antibiotikum gar nicht bzw. in einer Menge unterhalb der Nachweisgrenze vom Mikroorganismus hergestellt wird.

Die Kultivierung des Mikroorganismus erfolgt aerob, also beispielsweise submers unter Schütteln oder Rühren in Schüttelkolben oder Fermentern oder auf Festmedium, gegebenenfalls unter Einführen von Luft oder Sauerstoff. Sie kann in einem Temperaturbereich von etwa 15 bis 35°C, vorzugsweise bei etwa 25 bis 32°C, insbesonders bei 27 bis 30°C durchgeführt werden. Der pH-Bereich sollte zwischen 4 und 10 liegen, vorzugsweise zwischen 6.5 und 7.5. Man kultiviert den Mikroorganismus unter diesen Bedingungen im allgemeinen über einen Zeitraum von 48 bis 720 Stunden, vorzugsweise 72 bis 320 Stunden. Vorteilhaft kultiviert man in mehreren Stufen, d.h. man stellt zunächst eine oder mehrere Vorkulturen in einem flüssigen Nährmedium her, die dann in das eigentliche Produktions-medium, die Hauptkultur, beispielsweise im Volumenverhältnis 1:10-1:100, überimpft werden. Die Vorkultur erhält man z.B., indem man das Mycel in eine Nährlösung überimpft und etwa 20 bis 120 Stunden, bevorzugt 48 bis 72 Stunden, wachsen läßt. Das Mycel kann beispielsweise erhalten werden, indem man den Stamm etwa 1 bis 40 Tage, bevorzugt 14 bis 21 Tage, auf einem festen oder flüssigen Nährboden, beispielsweise Hefe-Malz-Glukose-Agar, Haferflocken-Agar oder Stärke-Agar, wachsen läßt.

Der Fermentationsverlauf und die Bildung der erfindungsgemäßen Antibiotika kann entsprechend dem Fachmann bekannten Methoden, wie z.B. durch Austestung der biologischen Aktivität in Bioassays oder durch chromatographische Methoden wie Dünnschichtchromatographie (DC) öder Hochleistungsflüssigkeitschromatographie (HPLC)

verfolgt werden.

Der *Actinomycetales* sp. DSM 14865 kann die erfindungsgemäßen Verbindung durch Submersfermentation bilden. Die erfindungsgemäßen Verbindungen können sowohl im Mycel als auch im Kulturfiltrat vorkommen, gewöhnlich befindet sich die Hauptmenge im Kulturfiltrat. Es ist deshalb zweckmäßig, die Fermentationslösung durch Filtration oder Zentrifugation zu trennen. Das Filtrat wird mit einem Adsorptionsharz als feste Phase extrahiert. Das Mycel, aber auch die Oberflächenkultur wird zweckmäßiger Weise mit einem geeigneten Lösungsmittel extrahiert, beispielsweise Methanol oder Propanol-2.

Die Extraktion kann in einem weiten pH-Bereich durchgeführt werden, es ist jedoch zweckmäßig, im neutralen oder schwach sauren Milieu, vorzugsweise zwischen pH 3 und pH 7 zu arbeiten. Die Extrakte können z.B. im Vakuum konzentriert und getrocknet werden.

Eine Methode der Isolierung des erfindungsgemäße Antibiotikums ist die Lösungsverteilung in an sich bekannter Weise.

Eine andere Methode der Reinigung ist die Chromatographie an Adsorptionsharzen wie z.B. an Diaion® HP-20 (Mitsubishi Casei Corp., Tokyo, Japan), an Amberlite® XAD 7 (Rohm and Haas, USA), an Amberchrom® CG (Toso Haas, Philadelphia, USA) oder an ähnlichen. Geeignet sind darüber hinaus zahlreiche Reverse-Phase Träger, z.B. $RP_8$ und $RP_{18}$, wie sie z.B. im Rahmen der HochdruckflüssigkeitsChromatographie (HPLC) allgemein bekannt geworden sind.

Eine weitere Reinigungsmöglichkeit für das erfindungsgemäße Antibiotikum besteht in der Verwendung von sog. Normal-Phasen-Chromatographie-Trägern, wie z.B. Kieselgel oder $Al_2O_3$ oder anderen in an sich bekannter Weise.

Ein alternatives Isolierungsverfahren ist die Verwendung von Molekularsieben, wie z.B. Fractogel® TSK HW-40 (Merck KGaA, Darmstadt, Deutschland), Sephadex® G-25 (Amersham Biosciences, Uppsala, Schweden) und andere, in an sich bekannter Weise. Es ist darüber hinaus auch möglich, aus angereichertem Material die neuen Polyencarbonsäuren durch Kristallisation zu gewinnen. Geeignet hierzu sind z.B. organische Lösungsmittel und ihre Gemische, wasserfrei oder mit Wasserzusatz oder verschiedene Salze. Ein zusätzliches Verfahren zur Isolierung und Reinigung der erfindungsgemäßen Antibiotika besteht in der Verwendung von Anionenaus-tauschern, vorzugsweise im pH-Bereich von 4 bis 10. Besonders geeignet ist hierfür die Verwendung von Pufferlösungen, denen man Anteile von organischen Lösungsmitteln hinzugefügt hat.

Es wurde überraschend gefunden, daß die erfindungsgemäßen Verbindungen starke Inhibitoren der Glycosyltransferase sind. Selektive und starke Hemmstoffe der Glycosyltransferase, wie zum Beispiel die Antibiotika der Moenomycingruppe, weisen stets antibakterielle Wirkungen auf, wobei eine geringe Inhibitor-Konzentration ($IC_{50}$) mit starker bakteriostatischer Aktivität einhergeht. Die erfingungsgemäßen Verbindungen wirken bakteriostatisch weil sie das Wachstum und Vermehrung der Bakterien hemmen, sie eignen sich daher zur Therapie von Erkrankungen, die durch bakterielle Infektionen verursacht werden. Tabelle 1 faßt die Inhibitor-Konzentrationen ($IC_{50}$) von einigen erfindungsgemäßen Verbindungen beispielhaft zusammen. Um spezifische und sehr wirksame Glycosyltransferase-Inhibitoren zu finden, wurde ein spezielles Testsystem angewandt. Hierbei wird ein Glycosyltransferase / $^3$H-Moenomycin-Komplex eingesetzt und mit zu prüfenden Substanzen zusammengebracht. Die Bestimmung der GT-inhibitorischen Wirkung beruht auf dem Verdrängen von radioaktiv markiertem Moenomycin A aus dem Glycosyltransferase (PBP 1b) / $^3$H-Moenomycin-Komplex, wobei das freigesetzte $^3$H-Moenomycin als lösliche Verbindung vom fixierten Komplex abgetrennt und die verbliebene Radioaktivität mit einem Geigerzähler gemessen werden kann.

Tabelle 1: Die Hemmkonstanten ($IC_{50}$-Werte) der erfindungsgemäßen Antibiotika im Glycosyltransferase Assay.

| | |
|---|---|
| Serpentemycin A: | 0.2 μM |
| Serpentemycin B: | 0.1 μM |

Zum Vergleich wurde die Hemmkonstante von Serpentene (Ritzau et al., Liebigs Ann. Chem. 1993, 433-435) bestimmt: der $IC_{50}$-Wert betrug 18 μM.

Die vorliegende Erfindung betrifft demzufolge auch die Verwendung einer Verbindung der Formel (I) als Arzneimittel, vorzugsweise zur Behandlung und/oder zur Prophylaxe von bakteriellen Infektionen.

Die Erfindung betrifft ferner sowie die Verwendung einer Verbindung der Formel (I) zur Herstellung eines Arzneimittels, vorzugsweise zur Behandlung und/oder zur Prophylaxe von bakteriellen Infektionen.

Des weiteren betrifft die vorliegende Erfindung ein Arzneimittel mit einem Gehalt an einer oder mehreren der erfindungsgemäßen Verbindungen.

Das besagte Arzneimittel wird durch Mischung von mindestens einer Verbindung der Formel (I) mit einem oder mehreren physiologischen geeigneten Hilfsstoffen hergestellt und in eine geeignete Darreichungsform gebracht.

Die erfindungsgemäßen Arzneimittel werden im allgemeinen oral, lokal oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei

deren Her-stellung üblicherweise physiologisch geeignete Hilfsmittel wie sprung-, Binde-, Überzugs-, Quellungs-, Gleit- oder Schmiermittel, Geschmacksstoffe, Süßungsmittel oder Lösungsvermittler Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Als zweckmäßige Dosierung werden 0.1 - 1000, vorzugsweise 0.2 - 100 mg/kg Körpergewicht verabreicht. Sie werden zweckmäßig in Dosierungseinheiten verabreicht, die mindestens die wirksame Tagesmenge der erfindungsgemäßen Verbindungen, z.B. 30 - 3000, vorzugsweise 50 - 1000 mg enthalten.

Die folgenden Beispiele sollen der näheren Erläuterung der Erfindung dienen, ohne die Breite der Erfindung in irgendeiner Weise zu begrenzen.

Beispiel 1 Herstellung einer Glycerinkultur von *Actinomycetales* sp. DSM 14865

30 ml Nährlösung (Malzextrakt 1,0%, Hefeextrakt 0,4 %, Glucose 0,4 %, pH 7,0 in Wasser) in einem sterilen 100 ml Erlenmeyerkolben wurden mit dem Stamm *Actinomycetales* sp, DSM 14865, beimpft und 7 Tage bei 28° C und 180 UpM auf einer rotierenden Schüttelmaschine inkubiert. Je 1,5 ml dieser Kultur wurden anschließend mit 2,5 ml 80 %-igem Glycerin verdünnt und bei -135°C gelagert.

Beispiel 2 Herstellung einer Vorkultur im Erlenmeyerkolben von *Actinomycetales* sp, DSM 14865

Je 100 ml Nährlösung (Glukose 1,5 %, Sojamehl 1,5 %, Cornsteep 0,5 %, $CaCO_3$ 0,2 % und NaCl 0,5 % pH 7,0) wurden in einem sterilen 300 ml Erlenmeyerkolben mit dem Stamm *Actinomycetales sp,* DSM 14865, beimpft und 4 Tage bei 28° C und 180 UpM auf einer rotierenden Schüttelmaschine inkubiert. Diese Vorkultur wurde anschließend zum Beimpfen der Hauptkulturen verwendet.

Beispiel 3 Bildungskinetik der aromatischen Polyencarbonsäuren der Schüttelkulturen des *Actinomycetales* sp, DSM 14865.

Es wurden Kulturen des *Actinomycetales* sp, DSM 14865 in 20 Erlenmeyerkolben, wie in Beispiel 2 beschrieben, angesetzt und die Kulturen auf einer Schüttelmaschine bis über einen Zeitraum von 2 Wochen inkubiert. Nach 48, 72, 96, 102, 120, 240 und 312 Stunden wurden Schüttelkolben entnommen. Nach Abtrennen des Mycels, Festphasenextraktion der aromatischen Polyencarbonsäuren aus dem Kulturfiltrat mit MCI-Gel® (Mitsubishi Chemical Industries) und Elution mit Methanol wurden die Extrakte durch HPLC, wie in Beispiel 8 beschrieben, untersucht. In der folgenden Tabelle sind die Veränderungen der HPLC-Flächeneinheiten in Abhängigkeit von der Inkubationszeit aufgeführt. Die Fächeneinheiten sind proportional zu den Konzentrationen der Produkte.

Tabelle 2: Veränderung der Produktkonzentrationen mit der Inkubationszeit der *Actinomycetales* sp, DSM 14865, Schüttelkulturen. Die Produktkonzentrationen sind proportional zu den angegebenen HPLC-Flächeneinheiten.

| Inkubationszeit | Serpentemycine | | Serpentene |
|---|---|---|---|
| | A | B | |
| 48h | 174 | 132 | 574 |
| 72h | 313 | 284 | 1948 |
| 96h | 727 | 443 | 2530 |
| 102h | 698 | 424 | 1878 |
| 120h | 816 | 470 | 1212 |
| 240h | 917 | 1216 | 47 |
| 312h | 882 | 1411 | 35 |

Während Serpentene bereits nach 96 Stunden das Bildungsmaximum erreicht hat, sind die höchsten Konzentrationen des Serpentemycins A erst nach 10 Tagen zu beobachten. Serpentemycin B liegt sogar nach 13 Tagen in den höchsten Konzentrationen vor.

Beispiel 4 Fermentation des *Actinomycetales* sp. DSM 14865 zur Produktion von Serpentemycin A.

Die Fermentation des Stammes *Actinomycetales* sp. DSM 14865 zur Produktion der erfindungsgemäßen Antibiotika erfolgte in 30 L Fermentern. Als Nährlösung diente: 10 g/L lösliche Stärke; 10 g/L Glucose; 10 g/L Glycerin; 2.5 g/L Cornsteep, flüssig; 5 g/L Pepton; 2 g/L Hefe-extrakt; 1 g/L NaCl; 3 g/L $CaCO_3$ in Wasser; pH, vor dem Sterilisieren, 7.2. In diesem Nährmedium wurde gutes Wachstum und schnelle Produktivität beobachtet. Die Fermentation wurde unter folgenden Bedingungen durchgeführt:

Sterilisation : in-situ, 20 min bei T=121 °C
Inokulum: 6 %
Rührgeschwindigkeit: 180 Upm
T: 28°C
Luft: 0,45 $m^3/h$
$pO_2$: keine Regelung,
pH, während der Fermentation: etwa 6,2; ohne Regelung.

Unter diesen Bedingungen zeigte sich ein gutes Wachstum innerhalb der ersten 70h, zu diesem Zeitpunkt war die C-Quelle im Medium verbraucht und das Wachstum stagnierte. Nach 80 h konnte eine erste Produktbildung der erfindungsgemäßen Verbindungen nachgewiesen werden. Die Fermenter wurde nach der 98 h abge-erntet.

Beispiel 5: Isolierung des Serpentemycin A aus der Kulturlösung des *Actinomycetales* sp. DSM 14865.

Nach Beendigung der Fermentation von *Actinomycetales* sp. DSM 14865 wurden 27.5 Liter Kulturbrühe des Fermenters, gewonnen nach Beispiel 4, unter Zusatz von ca. 2 % Filterhilfsmittel (Celite®) filtriert und die Zellmasse (1890 g) mit 6 Liter Methanol extrahiert. Die Wirkstoffhaltige, methanolische Lösung wurde durch Filtration vom Mycel befreit und im Vakuum konzentriert. Das Konzentrat wurde mit 25 L des Kulturfiltrates vereinigt, der pH-Wert auf 7 eingestellt und auf eine vorbereitete, 3 Liter ®MCI GEL, CHP20P-Säule aufgetragen. Eluiert wurde mit einem Gradienten von 50 mM $NH_4$-Acetat-Puffer in Wasser nach Propanol-2. Der Säulendurchfluß (7 Liter pro Stunde) wurde fraktioniert aufgefangen (je 2 Liter) und die Antibiotika enthaltenden Fraktionen 4 bis 11 sowie 16 bis 19 jeweils vereinigt und im Vakuum konzentriert. Mittels HPLC werden die Fraktionen untersucht. Die Fraktion 4 bis 11 beinhaltet die polareren Serpentemycine, die Fraktion 16 bis 19 das Serpentene. Die Serpenteneenthaltenden Fraktionen wurden auf LiChrospher® 100 RP-18e, 250-25, HPLC-Säulen zweimal chromatographisch gereinigt, zuerst mit 50 mM $NH_4$-Acetat-Puffer /Acetonitril-Gemischen, dann mit 0.05% Trifluoressig-säure / Acetonitrilgradienten. Die Gefriertrocknung der reinen Antibiotikum enthaltenden Fraktionen ergab 19 mg reines Serpentene.

Die polareren Verbindungen der Fraktionen 4 bis 11 wurden, nachdem sie im Vakuum eingeengt worden sind, erneut auf 160 mL MCI GEL® CHP20P säulenchromatographisch (Säulenmaße : 26 mm x 300 mm) im Gradientenverfahren gereinigt. Der Gradient reichte von 10% bis 60% Acetonitril in 50 mM $NH_4$-AcetatPuffer in 2 Stunden. Der Säulendurchfluß betrug 25 mL pro Minute, die Fraktionengröße 50 mL. In den Fraktionen 15 bis 35 befand sich die Verbindung Serpentemycin B und D, die Fraktionen 36 bis 39 beinhalteten Serpentemycin C und die Fraktionen 40 bis 43 Serpentemycin A. Letztere wurden im Vakuum vom organischen Lösungsmittel befreit, mit 0.01% Ascorbinsäure versetzt und auf einer LiChrospher® 100 RP-18e, 250-25, HPLC-Säule isokratisch mit 45% Acetonitril in 0.05% Trifluoressigsäure chromatographisch gereinigt. Die Fraktionen, die reines Serpentemycin A enthielten (Fr. 31 bis 35) wurden unter Lichtausschluß gefriergetrocknet und in Argon-Atmosphäre luftdicht abgefüllt (37 mg). $ESI^-$-Massenspektrometrie: 321.4 (M - H), $ESI^+$-Massenspektrometrie: 305.3 (MH - $H_2O$). UV-Maxima: 315 und 355 nm. Die NMR-Daten sind in der Tabelle 3 aufgeführt, wobei die Bezifferung der C-Atome wie folgt vorgenommen wurde:

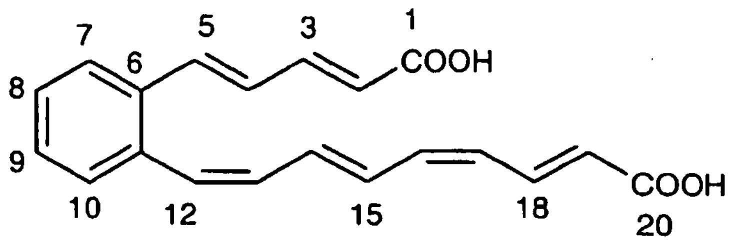

Tabelle 3: NMR-chemische Verschiebungen von Serpentemycin A in DMSO bei 300° K.

| | $^1H$ | $^{13}C$ |
|---|---|---|
| 1 | - | 167.43 |
| 2 | 6.02 | 122.54 |
| 3 | 7.35 | 144.35 |
| 4 | 7.04 | 128.09 |
| 5 | 7.19 | 137.16 |
| 6 | - | 134.33 |
| 7 | 7.73 | 125.93 |
| 8 | 7.35 | 127.90 |
| 9 | 7.35 | 128.57 |
| 10 | 7.27 | 130.22 |
| 11 | - | 135.78 |
| 12 | 6.81 | 130.33 |
| 13 | 6.63 | 131.24 |
| 14 | 6.59 | 132.61 |
| 15 | 7.15 | 130.33 |
| 16 | 6.41 | 136.57 |
| 17 | 6.21 | 127.45 |
| 18 | 7.70 | 138.40 |
| 19 | 5.95 | 123.07 |
| 20 | - | 167.48 |

Serpentemycin A:

Aussehen: Zitronengelbe, in mittelpolaren und polaren organischen Lösungsmitteln lösliche, in Wasser wenig lösliche Substanz. Stabil in neutralem und mild saurem Milieu, jedoch unbeständig in stark-saurer und stark-alkalischer Lösung.
Serpentemycin A ist licht- und luftempfindlich.
Summenformel: $C_{20}H_{18}O_4$
Molekulargewicht: 322.36

Beispiel 6: Isolierung und Beschreibung des Serpentemycin B.

Die Serpentemycin B-haltigen Fraktionen 36 bis 39 der 160 mL MCI GEL® CHP20P-Säule, gewonnen nach Beispiel 4, wurden mit im Vakuum eingeengt und die wässrige, noch etwas Acetonitril enthaltene Lösung auf eine LiChrospher® 100 RP-18e, 250-25, HPLC-Säule aufgetragen. Eluiert wurde isokratisch mit 42% Acetonitril in 0.05% Trifluoressigsäure (pH 2.4). Der Säulendurchfluß betrug 39 mL/Minute, es wurden Fraktionen je 19.5 mL abgenommen und mittels HPLC analysiert. Die Fraktionen 22 bis 24 enthielten das Antibiotikum Serpentemycin B, sie wurden auf der selben Säule rechromatographiert, wobei die Acetonitril-Konzentration im Elutionsmittel auf 40% reduziert wurde. Die Fraktionen 38 bis 42 enthielten reines Serpentemycin B, sie wurden gefriergetrocknet und ergaben 10.2 mg des Antibiotikums. $ESI^-$-Massenspektrometrie: 295.0985 (M - H), $ESI^+$-Massen-spektrometrie: 297.1164 (M + H), entsprechend der Summenformel $C_{18}H_{16}O_4$, UV-Maxima: 306 und 332 nm in Acetonitril/0.1 % Phosphorsäure in Wasser. Die NMR-Daten sind in der Tabelle 4 aufgeführt, die Bezifferung der Atome ist analog der Serpentemycin A-Numerierung.

Tabelle 4: NMR-chemische Verschiebungen von Serpentemycin B in DMSO bei 300° K.

| | $^1H$ | $^{13}C$ |
|---|---|---|
| 1 | - | 167.42 |
| 2 | 6.02 | 122.59 |
| 3 | 7.35 | 144.32 |
| 4 | 7.05 | 128.23 |
| 5 | 7.19 | 137.09 |
| 6 | - | 134.40 |
| 7 | 7.74 | 126.00 |
| 8 | 7.36 | 128.10 |
| 9 | 7.36 | 128.61 |
| 10 | 7.28 | 130.31 |
| 11 | - | 135.40 |
| 12 | 6.90 | 131.86 |
| 13 | 6.53 | 130.54 |
| 14 | 6.81 | 135.71 |
| 15 | 6.65 | 132.89 |
| 16 | 7.19 | 143.79 |
| 17 | 5.93 | 122.69 |
| 18 | - | 167.33 |

Serpentemycin B:

Aussehen: Hellgelbe, in mittelpolaren und polaren organischen Lösungsmitteln lösliche, in Wasser wenig lösliche Substanz. Stabil in neutralem und mild saurem Milieu, jedoch unbeständig in stark-saurer und stark-alkalischer Lösung.
Serpentemycin B ist licht- und luftempfindlich.
Summenformel: $C_{10}H_{16}O_4$
Molekulargewicht: 296.33

Beispiel 7: Hochdruckflüssigkeitschromatographie (HPLC) der Serpentemycine.

Die HPLC wurde unter folgenden Bedingungen durchgeführt:

| | |
|---|---|
| Säule: | Superspher 100 RP-18e®, 250-4, mit Vorsäule, |
| Mobile Phase: | 50 % Acetonitril in 0.1% Phosphorsäure, |
| Flußgeschwindigkeit: | 1 mL pro Minute, |
| Säulentemperatur: | 40° C, |

Detektion durch UV-Absorption bei 210 nm.

Folgende Retentionszeiten wurden beobachtet:

| | |
|---|---|
| Serpentemycin A | 10.1 Minuten, |
| Serpentemycin B | 5.6 Minuten |

Beispiel 8: Bestimmung der Glycosyltransferase-Hemmung durch Serpentemycine .

Der Assay wurde entsprechend Vollmer & Höltje (Antimicrobial Agents and Chemotherapy 2000, 44, 1181-1185) durchgeführt, wobei anstatt mit [3H]Benzylpenicillin m vorliegenden Beispiel mit gereinigtem Penicillin Binding Protein 1b (PBP 1b; 5 pM) durchgeführt, welches an der Glycosyltransferase-Stelle $^3$H-markiertes Moenomycin trägt. $^3$H-Moenomycin wird aus Moenomycin A (Kurz et al., Eur. J. Biochem., 1998, 252, 500-507) durch Hydrierung mit $^3H_2$ gewonnen:

In ein 1 $cm^3$ -Kolben wird 6 mg Moenomycin, gelöst in 300 $\mu$L Methanol, gegeben und 2 mg Palladium-Kohle (Degussa Type E10N/D) hinzugefügt. Dann wird unter luftausschluß mit 1 $cm^3$ $^3H_2$ begast und die Reaktionslösung 15 Minuten hydrieren lassen. Nach Beenden der Reaktion wird die Reaktionsmischung vom Katalysator des Glycosyltransferase / $^3$H-Moenomycin-Komplexes unmittelbar verwendet werden. Gesamt-Radioaktivität der Reaktionsproduktes: 6.56 GBq (177mCi), Spezifische Aktivität: 1.9 TBq/mmol. Der radioaktive Komplex wird an SPA PVT Copper His-Tag - Perlen fixiert. Es wird das durch die Inhibitoren verdrängte radioaktive Moenomycin gemessen.
SPA PVT Copper His-Tag - Perlen: Amersham RPN 0095;
PBS: GIBCO BRL 14200-067;
BSA: Calbiochem 12657;
NOG: SIGMA O-8001 (n-Octyl ß-D-glucopyranosid);
Tween 20: Acros 23336-067;
MikrotiterPlatten: Greiner Labortechnik;

Die Bestimmung wurde in Mikrotiterplatten durchgeführt. In die Mikrotiterplatten wurden 10 $\mu$L der Testlösung gegeben und 10 $\mu$L $^3$H-Moenomycin (25 nM, 3.1 kBq/Well) sowie 40 $\mu$L SPA-Perlen, beladen mit PBP 1b, (100 $\mu$g Perlen, 45 nM Enzym) versetzt. Die Platten wurden verschlossen und 8 Stunden bei Raumtemperatur stehen gelassen. Danach wurden die Perlen durch 3-minütiges Zentrifugieren bei 1300 Rpm von der Testlösung getrennt und die Verteilung der Radioaktivität einem WALLAC MicroBeta® 1450 Counter gemessen.

Die Berechnung der Hemmwerte erfolgte nach der Formel:

$$[1 - (cpm_{sample} - cpm_{low\ ctr})/(cpm_{high\ ctr} - cpm_{low\ ctr})] \times 100\ (\%).$$

**Patentansprüche**

1. Verbindung der Formel (IV)

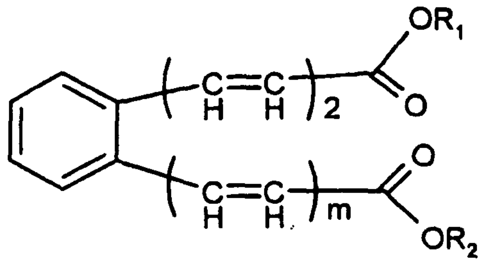

(IV)

wobei
$R_1$ H oder $C_1$-$C_6$-Alkyl,
$R_2$ H oder $C_1$-$C_6$-Alkyl, und
m 3 oder 4 ist, sowie deren physiologisch verträgliche Salze.

2. Verbindung der Formel (IV) gemäß Anspruch 1, wobei mindestens eine Polyengruppe mindestens eine *cis*-Doppelbindung enthält.

3. Verbindung der Formel (IV) gemäß einem oder mehreren der Ansprüche 1

oder 2, **gekennzeichnet durch** eine Verbindung der Formel (V)

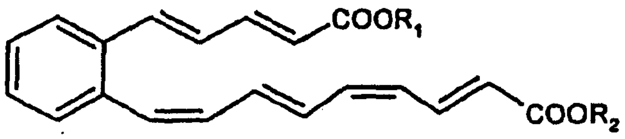

(V).

4. Verbindung der Formel (V) gemäß Anspruch 3, wobei $R_1$ und $R_2$ gleich H sind.

5. Verbindung der Formel (IV) gemäß einem oder mehreren der Ansprüche 1 oder 2, **gekennzeichnet durch** eine Verbindung der Formel (VI)

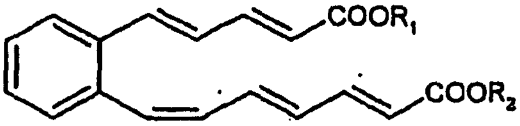

(VI).

6. Verbindung der Formel (VI) gemäß Anspruch 5, wobei $R_1$ und $R_2$ gleich H sind.

7. Verfahren zur Herstellung einer Verbindung der Formel (IV) gemäß Anspruch 1, **dadurch gekennzeichnet, daß**

   1. der Mikroorganismus *Actinomycotales* sp: DSM 14865 bzw. einer ihrer Varianten und/oder Mutanten in einem wäßrigen Nährmedium kultiviert wird, bis sich eine oder mehrere der Verbindungen Serpentemycin A

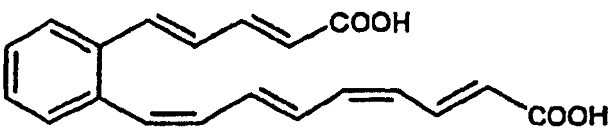

   und/oder Serpentemycin

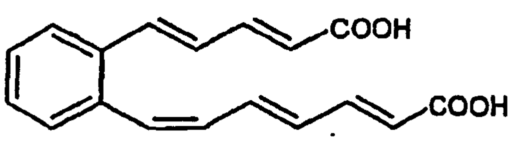

   in der Kulturbrühe anhäuft,
   2. Serpentemycin A oder B isoliert und gereinigt wird,
   3. Serpentemycin A oder B gegebenenfalls mit einem Alkylierungsmittel in eine Verbindung der Formel (IV) überführt wird,
   4. und die Verbindung der Formel (IV) gegebenenfalls in ein pharmakologisch verträgliches Salz überführt wird.

8. Verfahren gemäß Anspruch 7, **dadurch gekennzeichnet, daß** die Fermentation unter aeroben Bedingungen bei einer Temperatur zwischen 20 und 35°C und bei einem pH zwischen 4 und 10 durchgeführt wird.

9. Verwendung einer Verbindung gemäß einem oder mehreren der Ansprüche 1 bis 6 zur Herstellung eines Arzneimittels.

**10.** Verwendung einer Verbindung gemäß Anspruch 9 zur Herstellung eines Arzneimittels zur Behandlung und/oder Prophylaxe von bakteriellen Infektionskrankheiten.

**11.** Arzneimittel mit einem Gehalt an mindestens einer Verbindung gemäß einem oder mehreren der Ansprüche 1 bis 6 und einem oder mehreren physiologisch geeigneten Hilfsstoffen.

**12.** Verfahren zur Herstellung eines Arzneimittels gemäß Anspruch 11, **dadurch gekennzeichnet, daß** mindestens eine Verbindung gemäß einem oder mehreren der Ansprüche 1 bis 6 mit einem oder mehreren physiologisch geeigneten Hilfsstoffen in eine geeignete Darreichungsform gebracht wird.

**13.** Mikroorganismus *Actinomycetales* sp., DSM 14865.

## Claims

**1.** A compound of the formula (IV)

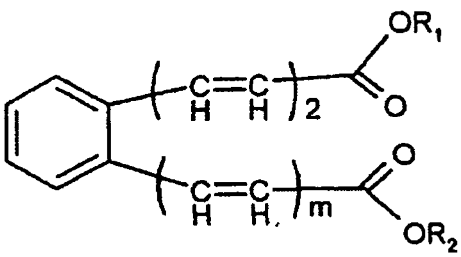

(IV)

where
$R_1$ is H or $C_1$-$C_6$-alkyl,
$R_2$ is H or $C_1$-$C_6$-alkyl, and
m is 3 or 4, and the physiologically tolerated salts thereof.

**2.** A compound of the formula (IV) as claimed in claim 1, wherein at least one polyene group contains at least one *cis* double bond.

**3.** A compound of the formula (IV) as claimed in one or more of claims 1 and 2, which is a compound of the formula (V)

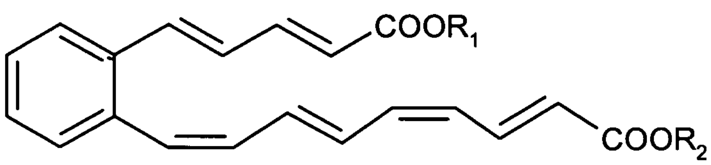

(V) .

**4.** A compound of the formula (V) as claimed in claim 3, where $R_1$ and $R_2$ are H.

**5.** A compound of the formula (IV) as claimed in one or more of claims 1 and 2, which is a compound of the formula (VI)

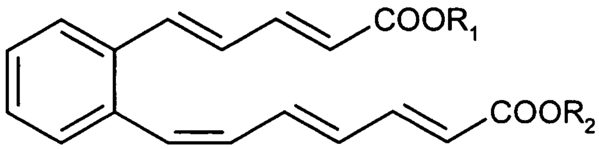

(VI).

6. A compound of the formula (VI) as claimed in claim 5, where $R_1$ and $R_2$ are H.

7. A process for preparing a compound of the formula (IV) as claimed in claim 1, which comprises

1. culturing the microorganism *Actinomycetales* sp.
DSM 14865, or one of its variants and/or mutants, in an aqueous nutrient medium until one or more of the compounds serpentemycin A

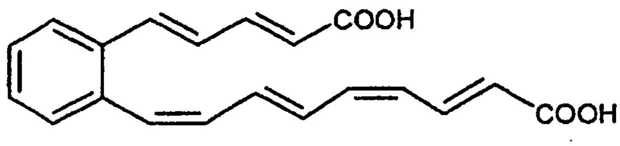

and/or serpentemycin B

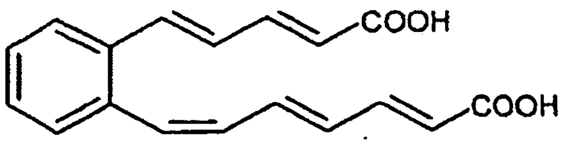

accrues in the culture broth,
2.isolating and purifying serpentemycin A or B,
3.where appropriate, using an alkylating agent to
convert serpentemycin A or B into a compound of the formula (IV),
4.and, where appropriate, converting the compound
of the formula (IV) into a pharmacologically tolerated salt.

8. A process as claimed in claim 7, wherein the fermentation is carried out under aerobic conditions at a temperature of between 20 and 35°C and at a pH between 4 and 10.

9. The use of a compound as claimed in one or more of claims 1 to 6 for producing a pharmaceutical.

10. The use of a compound as claimed in claim 9 for producing a pharmaceutical for the treatment and/or prophylaxis of infectious bacterial diseases.

11. A pharmaceutical having a content of at least one compound as claimed in one or more of claims 1 to 6 and one or more physiologically suitable auxiliary substances.

12. A process for producing a pharmaceutical as claimed in claim 11, which comprises bringing at least one compound as claimed in one or more of claims 1 to 6, together with one or more physiologically suitable auxiliary substances, into a suitable form for administration.

13. The microorganism *Actinomycetales* sp., DSM 14865.

## Revendications

1. Composé de formule (IV)

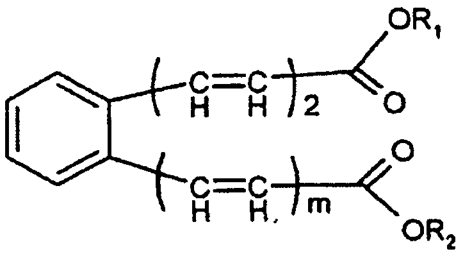

(IV)

dans laquelle
$R_1$ est H ou un radical alkyle en $C_1$-$C_6$,
$R_2$ est H ou un radical alkyle en $C_1$-$C_6$, et
m vaut 3 ou 4, ainsi que ses sels physiologiquement acceptables.

2. Composé de formule (IV) selon la revendication 1, dans lequel au moins un groupe polyène contient au moins une double liaison en *cis.*

3. Composé de formule (IV) selon l'une ou plusieurs des revendications 1 ou 2, **caractérisé en ce qu'**il s'agit d'un composé de formule (V)

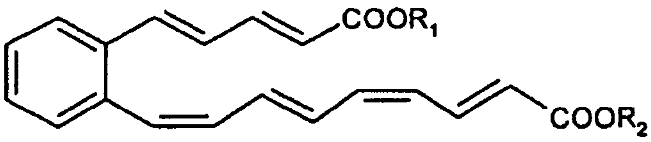

(V) .

4. Composé de formule (V) selon la revendication 3, dans lequel $R_1$ et $R_2$ sont H.

5. Composé de formule (IV) selon l'une ou plusieurs des revendications 1 ou 2, **caractérisé en ce qu'**il s'agit d'un composé de formule (VI)

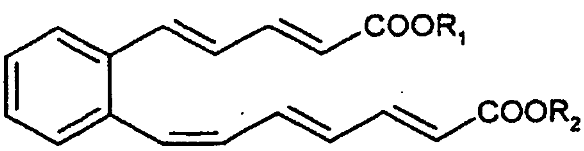

(VI) .

6. Composé de formule (VI) selon la revendication 5, dans lequel $R_1$ et $R_2$ sont H.

7. Procédé de préparation d'un composé de formule (IV) selon la revendication 1, **caractérisé en ce que**

   1. on cultive le microorganisme *Actinomycetales* sp. DSM 14865, ou l'un de ses variants et/ou mutants dans un milieu nutritif aqueux, jusqu'à ce qu'un ou plusieurs des composés serpentémycine A

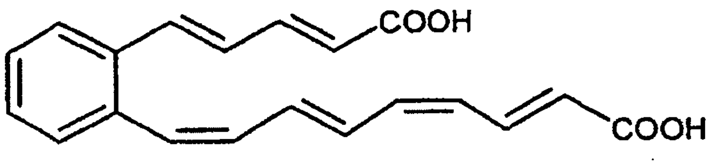

et/ou serpentémycine B

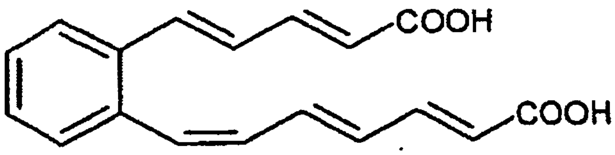

s'accumulent dans le bouillon de culture,
2. on isole et purifie la serpentémycine A ou B,
3. on convertit la serpentémycine A ou B, éventuellement avec un agent d'alkylation, en un composé de formule (IV),
4. et on convertit éventuellement le composé (IV) en un sel acceptable d'un point de vue pharmacologique.

**8.** Procédé selon la revendication 7, **caractérisé en ce que** la fermentation est mise en oeuvre dans des conditions aérobies à une température de 20 à 35°C et à un pH compris entre 4 et 10.

**9.** Utilisation d'un composé selon l'une ou plusieurs des revendications 1 à 6 pour préparer un médicament.

**10.** Utilisation d'un composé selon la revendication 9 pour préparer un médicament destiné au traitement et/ou à la prophylaxie de maladies infectieuses bactériennes.

**11.** Médicament contenant au moins un composé selon l'une ou plusieurs des revendications 1 à 6 et un ou plusieurs adjuvants physiologiquement appropriés.

**12.** Procédé de préparation d'un médicament selon la revendication 11, **caractérisé en ce qu'**on met sous une forme galénique appropriée au moins un composé selon l'une ou plusieurs des revendications 1 à 6, avec un ou plusieurs adjuvants appropriés d'un point de vue physiologique.

**13.** Le microorganisme *Actinomycetales* sp., DSM 14865.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **Ritzau et al.** *Liebigs Ann. Chem.,* 1993, 433-435
- **Kurz et al.** *Eur. J. Biochem.,* 1998, vol. 252, 500-507
- **Goldman ; Gange.** *Current Medicinal Chem.,* 2000, vol. 7, 801-820
- **Vollmer ; Höltje.** *Antimicrobial Agents and Chemotherapy,* 2000, vol. 44, 1181-1185
- **Darby et al.** *J. Org. Chem.,* 1977, vol. 42, 1960-1967
- **Jerry March.** Buch Advanced Organic Chemistry. John Wiley & Sons, 1992
- Remingtons Pharmaceutical Sciences. 1985, 1418
- **Brock et al.** Biology of Microorganisms. Prentice Hall, 1984, 238-247
- **H. Stolp.** Microbial ecology: organismus, habitats, activities. Cambridge University Press, 1988, 180